# EUROPÄISCHE PATENTSCHRIFT

(11) **EP 0 820 973 B1**
(45) Veröffentlichungstag und Bekanntmachung des Hinweises auf die Patenterteilung: **06.10.1999**
(21) Anmeldenummer: 97111018.4
(22) Anmeldetag: 02.07.1997
(51) Int. Cl.: C07C 11/02, C07C 31/125, C07C 53/126

(54) **Verfahren zur Herstellung von Buten-Oligomeren aus Feldbutanen**
Process for the preparation of butene oligomers from natural butanes
Procédé pour la préparation d'oligomères de butène à partir de butane naturel

(30) Priorität: 24.07.1996 DE 19629903
(43) Veröffentlichungstag der Anmeldung: 28.01.1998
(73) Patentinhaber: HÜLS AKTIENGESELLSCHAFT, 45764 Marl (DE)
(72) Erfinder: Nierlich, Franz, Dr., 45768 Marl (DE); Olbrich, Paul, Dr., 45721 Haltern (DE); Droste, Wilhelm, Dr., 45770 Marl (DE); Müller, Richard, Dr., 45770 Marl (DE); Toetsch, Walter, Dr., 45770 Marl (DE)

(56) Entgegenhaltungen:
- GB-A- 2 070 638
- US-A- 4 393 259
- US-A- 4 982 031

## Beschreibung

Die Erfindung betrifft ein Verfahren zur Herstellung von Butenoligomeren, die wertvolle Ausgangsstoffe für Weichmacheralkohole sind, aus Feldbutanen. Bevorzugte Butenoligomere sind die isomeren Octene, die dimere Butene sind und daher auch als Dibuten bezeichnet werden. Ein besonders gefragtes Dibuten ist Di-n-buten. Die Erfindung betrifft daher auch ein Verfahren, bei dem Di-n-buten aus Dibuten abgetrennt wird. Schließlich betrifft die Erfindung ein Verfahren. das - neben höheren Butenoligomeren - als Dibuten ausschließlich Di-n-buten ergibt.

Dibuten ist ein Isomerengemisch, das neben höheren Butenoligomeren durch Dimerisierung und/oder Co-Dimerisierung von Butenen, d.h. von n-Buten und/oder iso-Buten, bei der Oligomerisierung von Butenen entsteht. Als Di-n-buten bezeichnet man das Dimerisierungsprodukt von n-Buten, d.h. des 1-Butens und/oder 2-Butens. Wesentliche Bestandteile des Di-n-butens sind 3-Methyl-2-hepten, 3,4-Dimethyl-2-hexen und in untergeordnetem Maße n-Octene. Di-iso-buten ist das Dimerengemisch, das durch Dimerisierung von iso-Buten entsteht. Di-iso-buten enthält stärker verzweigte Moleküle als Dibuten, und dieses ist wiederum stärker verzweigt als Di-n-buten.

Dibuten, Di-n-buten und Di-iso-buten sind Ausgangsstoffe für die Herstellung von isomeren Nonanolen durch Hydroformylierung und Hydrierung der so entstehenden C₉-Aldenyde. Ester dieser Nonanole, insbesondere die Phthalsäureester, sind Weichmacher, die in bedeutendem Umfang hergestellt und vor allen für Polyvinylchlorid verwendet werden. Nonanole aus Di-n-buten sind in höherem Maße geradkettig als Nonanole aus Dibuten, die wiederum weniger verzweigt sind als Nonanole aus Di-iso-buten. Ester von Nonanolen aus Di-n-buten haben wegen ihrer in höherem Maße geradkettigen Struktur anwendungstechnische Vorteile gegenüber Estern aus Nonanolen auf Basis Dibuten und Di-iso-buten und sind besonders gefragt.

Man kann Butene für die Dimerisierung z.B. aus dem C₄-Schnitt von Steamcrackern oder von FC-Crackern gewinnen. Dieser wird in der Regel aufgearbeitet, indem man zunächst 1,3-Butadien durch eine selektive Wäsche, z.B. mit N-Methylpyrrolidon, abtrennt, iso-Buten ist ein erwünschter und besonders wertvoller Bestandteil des C₄-Schnitts, weil es sich zu begehrten Produkten chemisch umsetzen läßt, z.B. mit iso-Butan zu hochoctanigem Isooctan oder mit Methanol zum Methyltert, butylether (MTBE), der als Zusatz zum Fahrbenzin dessen Octanzahl verbessert. Nach der Umsetzung des iso-Butens bleiben die n-Butene sowie n- und iso-Butan zurück. Der Anteil der n-Butene an den Spaltprodukten der Steamcracker oder der FC-Cracker ist jedoch verhältnismäßig gering, nämlich in der Größenordnung von knapp 10 Gewichtsprozent, bezogen auf das hauptsachliche Zielprodukt Ethylen. Ein Steamcracker mit der respektablen Kapazität von 600.000 t/Jahr Ethylen liefert also nur rund 60.000 t/Jahr n-Butene. Man könnte zwar deren Menge (und die der iso-Butene) erhöhen, indem man die rund 15.000 t/Jahr n- und iso-Butan dehydriert, die neben den n-Butenen anfallen. Das empfiehlt sich jedoch nicht, weil Dehydrieranlagen hohe Investitionskosten erfordern und für eine so kleine Kapazität unwirtschaftlich sind.

iso-Buten ist, wie gesagt, ein gefragtes Crackprodukt und steht daher für die Oligomerisierung in der Regel nicht zur Verfügung. Die Menge an n-Butenen, die ein Steamcracker oder ein FC-Cracker unmittelbar erzeugt, reicht jedoch nicht aus, um genügend Dibuten für eine Nonanol-Anlage zu erzeugen, deren Kapazität so groß ist, daß sie mit den bestehenden großen Anlagen zur Herstellung bedeutender Weichmacheralkohole, wie 2-Ethylhexanol, wirtschaftlich konkurrieren könnte. Man müßte also n-Butene aus verschiedenen Steamcrackern oder FC-Crackern sammeln und gemeinsam oligomerisieren, um den Bedarf einer großen Nonanol-Anlage an Dibuten zu decken. Dem steht aber entgegen, daß der Transport von Flüssiggasen teuer ist, nicht zuletzt wegen der erforderlichen aufwendigen Sicherheitsmaßnahmen.

Es wäre daher erwünscht, wenn man Butene an nur einem Ort ohne Transport über größere Entfernungen in Mengen zur Oligomerisierung zur Verfügung stellen könnte, wie sie für den Betrieb einer großen Anlage zur Herstellung von Nonanolen, z.B. mit einer Kapazität von 200.000 bis 800.000 t/Jahr, erforderlich sind. Es wäre weiter erwünscht, ein Verfahren zur Herstellung von Butenoligomeren zu haben, bei dem man das wertvolle Di-n-buten aus dem Dibuten abtrennen kann. Schließlich wäre es erwünscht, wenn man das Verfahren so steuern könnte, daß neben höheren Butenoligomeren ausschließlich D-n-buten oder Di-iso-buten als Dibuten entsteht.

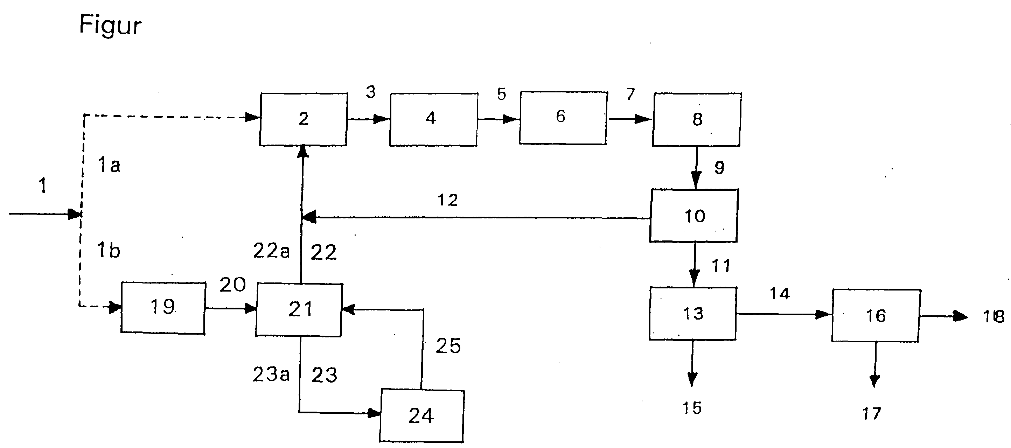

Das Verfahren nach der Erfindung wird durch das Blockschema der beigefügten Figur näher erläutert, in der die in der Folge näher beschriebenen Varianten A, B, C und D mit ihren obligatorischen und mit fakultativen Verfahrensstufen aufgeführt sind. Das Feldbutan 1 ist als Strom 1a den Varianten A, B und C zugeordnet, der alternative Strom 1b gehört zu den Variante D und E.

Die Erfindung ist dementsprechend ein Verfahren zur Herstellung von Buten-Oligomeren aus Feldbutanen, bei dem man
(a) die in den Feldbutanen 1 enthaltenen n- und iso-Butane in einer Dehydrierungsstufe 2 dehydriert und
(b) das Dehydrierungsgemisch (3) in einer Oligomerisierungsstufe (8) oligomerisiert, wobei man zwischen der Dehydrierungsstufe (2) und der Oligomerisiserungsstufe (8) eine Selektivhydrierung (4) und/oder eine Reinigungsstufe (6) mit Molekularsieb in beliebiger Reihenfolge anordnet.

Dieses Verfahren wird in der Folge als Variante A bezeichnet.

Bei einer bevorzugten, in der, Folge als Variante B bezeichneten Ausführungsform wird aus den Oligomeren 11, die nach der Abtrennung der Restgase 12 aus dem Oligomerisierungsgemisch 9 verbleiben, das Dibuten 14 abgetrennt,

Aus dem Dibuten 14 kann man bei einer anderen, in der Folge als Variante C bezeichneten bevorzugten Ausführungsform Di-n-buten 17 abtrennen,

Weiterhin steuert man bei einer anderen, besonders bevorzugten Ausführungsform, in der Folge Variante D genannt, das Verfahren so, daß neben höheren Butenoligomeren ausschließlich Di-n-buten 17 entsteht. indem man aus dem gegebenenfalls zuvor hydrierten Feldbutan 1 durch fraktionierte Destillation n-Butan 22 abtrennt, das verbleibende iso-Butan 23 in einer Isomerisierungsstufe 24 zu einem Gemisch von n-Butan und iso-Butan isomerisiert, aus dem Isomerisierungsgemisch 25 durch fraktionierte Destillation das n-Butan abtrennt und zusammen mit dem direkt aus dem Feldbutan 1 abgetrennten n-Butan 22 in die Dehydrierungsstufe 2 führt und das verbleibende iso-Butan 23 in die Isomerisierungsstufe 24 zurückführt.

Schließlich kann man in einer Variante E das Verfahren so steuern, daß neben höheren Butenoligomeren ausschließlich Di-iso-buten entsteht, indem man aus dem gegebenenfalls zuvor hydrierten Feldbutan 1 durch fraktionierte Destillation iso-Butan 22a abtrennt, das verbleibende n-Butan 23a in einer Isomerisierungsstufe 24 zu einem Gemisch von n-Butan und iso-Butan isomerisiert, aus dem Isomerisierungsgemisch 25 durch fraktionierte Destillation das iso-Butan abtrennt und zusammen mit dem direkt aus dem Feldbutan 1 abgetrennten iso-Butan 22a in die Dehydrierungsstufe 2 führt und das verbleibende n-Butan 23a in die Isomerisierungsstufe 24 zurückführt.

Das Verfahren nach der Erfindung mit seinen Varianten A bis E zeichnet sich durch hohe Flexibilität aus. Man kann also je nach den Erfordernissen des Marktes gewünschtenfalls ausschließlich Di-n-buten, Dibuten, nebeneinander Di-n-buten und andere Dibutene oder ausschließlich Di-iso-buten erzeugen, wenn auch letzteres wohl nur selten das gewünschte Hauptprodukt sein wird.

Als Feldbutane bezeichnet man die C₄-Fraktion der "feuchten" Anteile des Erdgases sowie der Erdölbegleitgase, die durch Abkühlung auf etwa -30°C in flüssiger Form aus den Gasen abgetrennt werden. Durch Tieftemperaturdestillation gewinnt man daraus die Feldbutane, deren Zusammensetzung je nach Lagerstätte schwankt, die jedoch im allgemeinen etwa 30% iso-Butan und 65% n-Butan enthalten. Weitere Bestandteile sind in der Regel etwa 2 % C_{<4}-Kohlenwasserstoffe und etwa 3 % C_{>4}-Kohlenwasserstoffe. Feldbutane können ohne Auftrennung als Einsatzstoffe in Steamcrackern oder als Zusatz zum Fahrbenzin verwendet werden. Sie lassen sich durch durch fraktionierte Destillation in n-Butan und iso-Butan zerlegen. Iso-Butan wird z.B. in bedeutendem Umfang für die Herstellung von Propylenoxid durch Co-Oxidation von Propylen und iso-Butan sowie als Alkylierungsmittel verwendet, mit dem n-Buten bzw. iso-Buten zu iso-Octan alkyliert, das wegen seiner hohen Octanzahl als Zusatz zum Fahrbenzin geschätzt wird, n-Butan hat dagegen nur weniger bedeutende Verwendungen gefunden. Es dient z.B. als Butangas zu Heizzwecken oder wird in vergleichsweise kleinen Mengen beispielsweise zur Herstellung von Polymeren oder Copolymeren oder von Maleinsäureanhydrid durch Luftoxidation verwendet. Früher wurde n-Butan auch über die Stufe des n-Butens zu 1,3-Butadien dehydriert, doch ist dieses Verfahren inzwischen unwirtschaftlich geworden.

In US 4 393 259 ist ein Verfahren zur Herstellung von C₈-Kohlenwasserstoffen beschrieben, wobei Reinstbutan bzw. Propan zunächst partiell dehydriert und anschließend zu C₈-Kohlenwasserstoffen kondensiert werden.

Weil iso-Butan der begehrtere Bestandteil des Feldbutans ist, wird n-Butan in großem Maßstab zu iso-Butan isomerisiert (vgl. z.B. R.A. Pogliano et al.. Dehydrogenation-based Ether Production, 1996 Petrochemicai Review, DeWitt & Company, Houston, Texas, Butamer^{(R)}-Verfahren, Seite 6; sowie S.T.Bakas, F.Nierlich et al., Production of Ethers from Field Butanes and Refinery Streams. AIChE Summer Meeting, 1990. San Diego, California, Seite 11). Es lag daher nicht im Trend der Technik, ein Verfahren zu entwickeln, das in den Varianten A, B und C auch und sogar gerade auf die Nutzung des n-Butans im Feldgas abzielt, aus dem über die Zwischenstufe Di-n-buten bevorzugte Nonanole hergestellt werden. Es läuft gegen den Trend der Technik, wenn in der Variante D sogar das üblicherweise begehrte iso-Butan zu n-Butan isomerisiert wird.

### Variante A

Die Feldbutane 1a werden zunächst in der Dehydrierungsstufe 2 dehydriert. Die Dehydrierung ist eine Co-Dehydrierung. Es ist bemerkenswert, daß die Dehydrierung des Feldbutans, das ein Gemisch aus Bestandteilen mit unterschiedlichem Dehydrierverhalten ist, so glatt gelingt. Die Verfahrensbedingungen entsprechen weitgehend denjenigen, wie sie für n- und iso-Butan oder andere niedere Kohlenwasserstoffe bekannt sind. So beschreiben S.T.Bakas, F.Nierlich et al., loc. cit., Seite 12 ff., das Oleflex^{(R)}-Verfahren, das allgemein für die selektive Herstellung von leichten Olefinen geeignet ist und mit dem iso-Butan mit einer Selektivität von 91 bis 93 % zu iso-Buten dehydriert werden kann. Weitere einschlägige Veröffentlichungen sind die von G.C.Sturtevant et al., Oleflex - Selective Production of Light Olefins, 1988 UOP Technology Conference sowie ER 0 149 698. Man führt die Dehydrierung zweckmäßig in der Gasphase an fest angeordneten oder fluidisierten Katalysatoren durch. z.B. an Chrom(III)-oxid oder vorteilhaft an Platinkatalysatoren mit Aluminiumoxid oder Zeolithen als Träger. Die Dehydrierung findet im allgemeinen bei Temperaturen von 400 bis 800°C, vorteilhaft von 550 bis 650°C statt. Man arbeitet in der Regel bei Atmosphärendruck oder leicht erhöhtem Druck bis zu 3 bar. Die Verweilzeit in der Katalysatorschicht liegt, je nach Katalysator, Temperatur und angestrebtem Umsatzgrad, im allgemeinen zwischen 1 und 60 Minuten. Der Durchsatz liegt dementsprechend in der Regel zwischen 0.6 und 36 kg Feldbutan je m³ Katalysator und Stunde.

Es ist zweckmäßig, die Dehydrierung nur soweit zu treiben, daß im Dehydrierungsgemisch 3 etwa 50% der n- und iso-Butane unverändert bleiben. Zwar kann man bei höherer Temperatur höhere Umsatzgrade erreichen. Dann laufen aber in steigendem Maße Crackreaktionen ab. die die Ausbeute vermindern und, infolge von Koksablagerungen, die Lebensdauer des Dehydrierungskatalysators herabsetzen. Die optimalen Kombinationen der Reaktionsbedingungen. die zu den gewünschten Umsatzgraden führen, wie Art des Katalysators, Temperatur und Verweilzeit, lassen sich unschwer durch orientierende Versuche ermitteln.

Das Dehydrierungsgemisch 3 enthält in der Regel 90 bis 95 % C₄-Kohlenwasserstoffe und daneben Wasserstoff sowie niedriger- und höhersiedende Anteile, die zum Teil aus dem Feldbutan 1 stammen, zum Teil in der Dehydrierungsstufe 2 entstanden sind. Es wird vor der Oligomerisierung zweckmäßig gereinigt. In einer ersten Reinigungsstufe (in der Figur nicht dargestellt) werden die C₄-Fraktion und die höhersiedenden Anteile herauskondensiert. Das Kondensat wird unter Druck destilliert, wobei mitkondensierte, gelöste C_{<4}-Kohlenwasserstoffe über Kopf gehen. Aus dem Sumpfprodukt gewinnt man in einer weiteren Destillation als Hauptprodukt die C₄-Kohlenwasserstoffe und als Rückstand die vergleichsweise kleine Menge an C_{>4}-Kohlenwasserstoffe.

Die C₄-Kohlenwasserstoffe enthalten je nach Umsatzgrad im allgemeinen kleine Mengen, wie 0,01 bis 5 Volumenprozent, 1,3-Butadien. Es ist empfehlenswert, diesen Bestandteil zu entfernen, da er selbst in deutlich geringeren Mengen den Oligomerisierungskatalysator schädigen kann. Ein geeignetes Verfahren ist die Selektivhydrierung 4, die zudem den Anteil des erwünschten n-Butens erhöht. Ein geeignetes Verfahren wurde z.B. von F.Nierlich et al. in Erdöl & Kohle, Erdgas. Petrochemie, 1986,, Seite 73 ff. beschrieben. Es arbeitet in flüssiger Phase mit vollständig gelöstem Wasserstoff in stöchiometrischen Mengen. Als selektive Hydrierkatalysatoren eignen sich z.B. Nickel und insbesondere Palladium auf einem Träger. z.B. 0,3 Gewichtsprozent Palladium auf Aktivkohle oder vorzugsweise auf Aluminiumoxid. Eine geringe Menge Kohlenmonoxid im ppm-Bereich fördert die Selektivität der Hydrierung des 1,3-Butadiens zum Monoolefin und wirkt der Bildung von Polymeren, dem sogenannten "green oil" entgegen, die den Katalysator inaktivieren. Das Verfahren arbeitet im allgemeinen bei Raumtemperatur oder erhöhten Temperaturen bis zu etwa 60°C und unter erhöhten Drücken, die zweckmäßig im Bereich von bis zu 20 bar liegen. Der Gehalt an 1,3-Butadien im C₄-Schnitt des Dehydrierungsgemisches wird auf diese Weise auf Werte von <1 ppm gesenkt.

Weiterhin ist es zweckmäßig, die nunmehr von 1,3-Butadien weitgehend befreite C₄-Fraktion des Dehydrierungsgemisches 5 vor der Oligomerisierungsstufe über die Reinigungsstufe 6, ein Molekularsieb, zu leiten, wodurch weitere für den Oligomerisierungskatalysator schädliche Stoffe entfernt werden und dessen Lebensdauer weiter erhöht wird. Zu diesen schädlichen Stoffen zählen Sauerstoff- und Schwefelverbindungen. Dieses Verfahren ist von F.Nierlich et al. in EP-B1 0 395 857 beschrieben worden. Man verwendet zweckmäßig ein Molekularsieb mit einem Porendurchmesser von 4 bis 15 Angström, vorteilhaft von 7 bis 13 Angström. In manchen Fällen ist es aus wirtschaftlichen Gründen zweckmäßig, das Dehydrierungsgemsch nacheinander über Molekularsiebe mit unterschiedlichen Porengrößen zu leiten. Das Verfahren kann in der Gasphase, in Flüssigphase oder in Gasflüssigphase durchgeführt werden. Der Druck beträgt dementsprechend im allgemeinen 1 bis 200 bar. Man arbeitet zweckmäßig bei Raumtemperatur oder erhöhten Temperaturen bis zu 200°C.

Die chemische Natur der Molekularsiebe ist weniger wichtig als ihre physikalische Beschaffenheit, d.h. insbesondere die Porengröße. Man kann also die verschiedensten Molekularsiebe einsetzen, sowohl kristalline, natürliche Aluminiumsilikate, z.B. Schichtgittersilikate, als auch synthetische Molekularsiebe, z.B. solche mit Zeolithstruktur. Zeolithe vom A-, X- und Y-Typ sind u.a. von Bayer AG, Dow Chemical Co. , Union Carbide Corporation. Laporte Industries Ltd. und Mobil Oil Co. erhältlich. Für das Verfahren eignen sich auch solche synthetische Molekularsiebe, die neben Aluminium und Silicium noch andere, durch Kationenaustausch eingeführte Atome enthalten, wie Gallium. Indium oder Lanthan sowie Nickel. Kobalt, Kupfer, Zink oder Silber, Weiterhin sind synthetische Zeolithe geeignet, bei denen neben Aluminium und Silicium noch andere Atome, wie Bor oder Phosphor, durch Mischfällung in das Gitter eingebaut worden sind.

Die Selektivhydrierungsstufe 4 und/oder die Reinigungsstufe 6 mit einem Molekularsieb gehören zu dem Verfahren nach der Erfindung. Deren Reihenfolge ist im Prinzip beliebi jedoch wird die in der Figur angegebene Reihenfolge bevorzugt.

Das gegebenenfalls auf die beschriebene Weise vorbehandelte Dehydrierungsgemisch 7 wird in die Oligomerisierungsstufe 8 geleitet, die ein wesentlicher Teil des Verfahrens nach der Erfindung ist. Die Oigomerisierung ist eine Co-Oligomerisierung von n-Butenen und iso-Buten, die in an sich bekannter Weise durchgeführt wird, wie z.B. von F. Nierlich in Oligomerization for Better Gasoline. Hydrocarbon Processing, 1992. Seite 45 ff., oder von F.Nierlich et al. in dem bereits erwähnten EP-B1 0 395 857 beschrieben wurde. Man arbeitet im allgemeinen in flüssiger Phase und wendet z.B. als homogenen Katalysator ein System an, das aus Nickel(II)-octoat. Ethylaluminiumchlorid und einer freien Fettsäure besteht (DE-PS 28 55 423), oder vorzugsweise einen der zahlreichen bekannten, fest angeordneten oder im, Oligomerisierungsgemisch suspendierten Katalysatoren auf Basis von Nickel und Silicium. Die Katalysatoren enthalten oftmals zusätzlich Aluminium. So beschreibt die DD-PS 160 037 die Herstellung eines Nickel und Aluminum enthaltenden Fällungskatalysators auf Siliciumdioxid als Trägermaterial. Andere brauchbare Katalysatoren erhält man, indem man auf der Oberfläche der Trägermaterialien befindliche positiv geladene Teilchen, wie Protonen oder Natriumionen, gegen Nickelionen austauscht. Dies gelingt bei den verschiedensten Trägermaterialien, wie amorphem Aluminiumsilikat (R.Espinoza et al., Appl .Kat., 31 (1987) Seiten 259-266; kristallinem Aluminiumsilikat (DE-PS 20 29 624; Zeolithen vom ZSM-Typ (NL-PS 8 500 459; einem X-Zeolith (DE-PS 23 47 235); X- und Y-Zeolithen (A.Barth et al., Z.Anorg.Allg.Chem. 521. (1985) Seiten 207-214); und einem Mordenit (EP-A-0 281 208).

Die Co-Oligomerisierung erfolgt zweckmäßig, je nach Katalysator, bei 20 bis 200°C und unter Drücken von 1 bis 100 bar. Die Reaktionszeit (oder Kontaktzeit) beträgt im allgemeinen 5 bis 60 Minuten. Die Verfahrensparameter, insbesondere die Art des Katalysators, die Temperatur und die Kontaktzeit, werden so aufeinander abgestimmt, daß der gewünschte Oligomerisierungsgrad erreicht wird. Im Falle der Nonanole als gewünschtem Zielprodukt ist das vorwiegend eine Dimersierung. Dazu darf man die Reaktion natürlich nicht auf vollen Umsatz fahren, sondern strebt zweckmäßig Umsätze von 30 bis 70 % pro Durchgang an. Die optimalen Kombinationen der Verfahrensparameter lassen sich durch orientierende Versuche ohne Schwierigkeiten ermitteln.

Aus dem Oligomerisierungsgemisch 9 wird in einer Trennstufe 10 das Restgas 12 abgetrennt und in die Dehydrierungsstufe 2 zurückgeführt. Wenn in der Oligomerisierungsstufe 8 ein Katalysator vom Typ der erwähnten flüssigen Katalysatoren verwendet wurde, sollte das Restgas 12 zuvor zur Schonung des Dehydrierungskatalysators gereinigt werden. Man behandelt zunächst das Oligomerisierungsgemisch mit Wasser, um die Katalysatorbestandteile zu extrahieren. Das abgetrennte Restgas 12 wird dann mit einem geeigneten Molekularsieb getrocknet, wobei auch andere Nebenbestandteile abgetrennt werden. Danach entfernt man durch selektive Hydrierung, z.B. an Palladiumkatalysatoren, mehrfach ungesättigte Verbindungen, wie Butine, und führt schließlich das so gereinigte Restgas 12 in die Dehydrierungsstufe 2 zurück. Diese Maßnahmen zur Reinigung des Restgases 12 erübrigen sich, wenn ein fester Oligomerisierungskatalysator verwendet wird.

Die nach Abrennung des Restgases 12 verbleibenden Oligomeren 11 eignen sich wegen ihrer verzweigten Komponenten als Zusatz zum Fahrbenzin zur Verbesserung der Octanzahl.

### Variante B

Die Oligomeren 11 werden in der Destillationsstufe 13 in Dibutene 14 sowie Trimere 15, d.h. isomere Dodecene, und noch höhere Oligomere getrennt, wobei die Hauptfraktion aus den erwünschten Dibutenen 14 besteht. Die Dodecene 15 können hydroformyliert, die Hydroformylierungsprodukte hydriert und die so erhaltenen Tridecanole oxethyliert werden, wodurch man wertvolle Waschrohstoffe erhält. Die Dibutene 14 sind unmittelbar als Ausgangsstoff für die Herstellung von Nonanol geeignet.

### Variante C

Wenn es auf die besonderen Eigenschaften der Nonanole aus Di-n-buten ankommt, werden die Dibutene 14 in der Feindestillationsstufe 16 in Di-n-buten 17 und die als stärker verzweigte Moleküle leichter siedenden restlichen Dibutene 18 getrennt, die ebenfalls für die Herstellung von Nonanolen verwendet oder dem Fahrbenzin zugesetzt werden können. Diese Verfahrensweise ist eine günstigere Alternative zu der Variante, bei der man aus dem Co-Dehydrierungsgemisch 7 durch Destillation n- und iso-Butene abtrennt und diese Isomeren getrennt oligomerisiert. Diese Variante würde zwei getrennte Oligomerisierungsstufen erfordern, die erheblich kapitalintensiver und auch im Betrieb aufwendiger wären als nur eine, wenn auch größere Co-Oligomerisierungsstufe 8 in Verbindung mit einer Feindestillationsstufe 16.

### Variante D

Diese Variante wird gewählt, wenn man ausschließlich Di-n-buten als Dibuten herstellen will. Wenn das Feldbutan 1b olefinisch ungesättigte Bestandteile enthält, leitet man es vorteilhaft zunächst in eine Hydrierstufe 19, weil diese Bestandteile die spätere Isomerisierung des iso-Butans stören können. Die Hydrierung erfolgt in an sich bekannter Weise, wie z.B. von K.H.Walter et al. in The Hüls Process for Selective Hydrogenation of Butadien in Crude C₄ 's. Development and Technical Application. DGKM-Tagung Kassel, November 1993, beschrieben. Man arbeitet also zweckmäßig in flüssiger Phase und, je nach Katalysator, bei Raumtemperatur oder erhöhter Temperatur von bis zu 90°C und einem Druck von 4 bis 20 bar, wobei der Partialdruck des Wasserstoffs 1 bis 15 bar beträgt. Man verwendet die für die Hydrierung von Olefinen üblichen Katalysatoren. z.B. 0,3 % Palladium auf Aluminiumoxid.

Die hydrierten Feldbutane 20 werden in die Trennstufe 21 geleitet. Diese besteht im allgemeinen aus einer gut wirksamen Kolonne, in der n-Butan 22 und iso-Butan 23 durch fraktionierte Destillation getrennt werden. Die Kolonne 21 wird in üblicher Weise betrieben, zweckmäßig unter einem Druck von 4 bis 7 bar. Die C_{>4}-Kohlenwasserstoffe fallen als Sumpfprodukt an, n-Butan 22 wird im Seitenstrom abgezogen und geht, zusammen mit dem Restgas 12, in die Dehydrierung 2 und das um 10 bis 20°C niedriger siedende iso-Butan 23 zusammen mit leichteren Enden in die Isomerisierungsstufe 24, in der iso-Butan maximal bis zu einem Gleichgewicht, das je nach Temperatur bei ca. 40 bis 55 % n-Butan und 45 bis 60 % iso-Butan liegt, in n-Butan umgewandelt wird. Die Isomerisierung von n- und iso-Butan ist eine bekannte Reaktion, wenn auch in der Regel mit dem Ziel. iso-Butan zu gewinnen (siehe z.B. H.W.Grote. Oil and Gas Journal. 56 (13, Seite 73 ff., (1958)). Man arbeitet im allgemeinen in der Gasphase zweckmäßig bei einer Temperatur von 150 bis 230°C, unter einem Druck von 14 bis 30 bar und mit einem Platin-Katalysator auf Aluminiumoxid als Träger, dessen Selektivität durch Dotierung mit einer Chlorverbindung, wie Tetrachlorkohlenstoff, noch verbessert werden kann. Man setzt vorteilhaft eine kleine Menge Wasserstoff zu, um einer Dehydrierung entgegenzuwirken. Die Selektivität der Isomerisierung zu n-Butan ist hoch. Crackung zu kleineren Bruchstücken findet nur in untergeordnetem Maße (ca. 2 %) statt.

Das Isomerisierungsgemisch 25 muß in die Isomeren getrennt werden. Dies geschieht zweckmäßig in der ohnehin vorhandenen Kolonne 21, von der n-Butan in die Dehydrierungsstufe 2 gelangt, die im Unterschied zu den Varianten A, B und C keine Co-Dehydrierungsstufe ist. Im weiteren Ablauf entspricht die Variante D den anderen Varianten. In der Oligomerisierungsstufe 8 läuft wiederum eine Co-Oligomerisierung ab. denn das n-Buten aus der Dehydrierungsstufe 2 ist ja ein Gemisch aus 1-Buten und 2-Buten. Die Feindestillationsstufe 16 kann allerdings entfallen, da das Dibuten 14 bereits Di-n-buten ist.

### Variante E

Diese Variante wählt man, wenn, wohl nur ausnahmsweise, ausschließlich Di-iso-buten als Dibuten gewünscht wird. Man verwendet dann die Anordnung der Variante D, leitet jedoch aus der Kolonne 21 iso-Butan 22a in die Dehydrierstufe 2, in der wiederum, wie in der Variante D und anders als bei den Varianten A, B und C, keine Co-Dehydrierung stattfindet. Das n-Butan 23a wird aus der Kolonne 21 in die Isomerisierungsstufe 24 geleitet und dort maximal bis zum Gleichgewicht zu iso-Butan isomerisiert. Dieses wird von n-Butan getrennt, wiederum zweckmäßig in der Kolonne 21, und ebenfalls in die Dehydrierungsstufe 2 geleitet, während das n-Butan in die Isomerisierungsstufe 24 zurückgeht. Auf diese Weise wird das n-Butan vollständig in iso-Butan umgewandelt. Das Dehydrierungsgemisch 3 wird zweckmäßig gerefnigt, wie bei der Variante A beschrieben. Die Oligomerisierung in der Oligomerisierungsstufe 8 ist eine Homooligomerisierung, weil nur iso-Butan daran beteiligt ist, und in der Destillationsstufe 13 fällt Di-iso-buten an. Die Feindestillation 16 entfällt ebenfalls.

## Patentansprüche

1. Verfahren zur Herstellung von Buten-Oligomeren aus Feldbutanen,
dadurch gekennzeichnet,
daß man
(a) die in den Feldbutanen (1) enthaltenen n- und iso-Butane in einer Dehydrierungsstufe (2) dehydriert und
(b) das Dehydrierungsgemisch (3) in einer Oligomerisierungsstufe (8) oligomerisiert, wobei man zwischen der Dehydrierungsstufe (2) und der Oligomerisierungsstufe (8) eine Selektivhydrierung (4) und/oder eine Reinigungsstufe (6) mit Molekularsieb in beliebiger Reihenfolge anordnet.

2. Verfahren nach Anspruch 1,
dadurch gekennzeichnet,
daß man aus dem Oligomerisierungsgemisch (9) das Restgas (12) abtrennt und, gegebenenfalls nach Reinigung, in die Dehydrierungsstufe (2) zurückführt.

3. Verfahren nach Anspruch 2,
dadurch gekennzeichnet,
daß man aus den nach Abtrennung des Restgases (12) verbliebenen Oligomeren (11) das Dibuten (14) abtrennt.

4. Verfahren nach Anspruch 3,
dadurch gekennzeichnet,
daß man die Dibutene (14) in einer Feindestillationsstufe (16) in Di-n-butene (17) und restliche Dibutene (18) trennt.

5. Verfahren nach einem der Ansprüche 1 bis 2,
dadurch gekennzeichnet,
daß man ausschließlich Di-n-buten erzeugt wird, indem man aus dem gegebenenfalls vorhydrierten Feldbutan (1) durch fraktionierte Destillation n-Butan (22) abtrennt und in die Dehydrierungsstufe (2) leitet, das verbleibende iso-Butan (23) in einer Isomerisierungsstufe (24) zu einem Gemisch von n-Butan und iso-Butan isomerisiert, aus dem Isomerisierungsgemisch (25) durch fraktionierte Destillation das n-Butan abtrennt und zusammen mit dem direkt aus dem Feldbutan abgetrennten n-Butan (22) in die Dehydrierungsstufe (2) führt sowie das verbleibende iso-Butan (23) in die Isomerisierungsstufe (24) zurückführt.

6. Verfahren nach einem der Ansprüche 1 bis 2,
dadurch gekennzeichnet,
daß ausschließlich Di-iso-buten erzeugt wird, indem man aus dem gegebenenfalls vorhydrierten Feldbutan (1) durch fraktionierte Destillation iso-Butan abtrennt und in die Dehydrierungsstufe (2) leitet, das verbleibende n-Butan in einer Isomerisierungsstufe (24) zu einem Gemisch von n-Butan und iso-Butan isomerisiert, aus dem Isomerisierungsgemisch (25) durch fraktionierte Destillation das iso-Butan abtrennt und zusammen mit dem direkt aus dem Feldbutan abgetrennten iso-Butan in die Dehydrierungsstufe (2) führt sowie das verbleibende n-Butan in die Isomerisierungsstufe (24) zurückführt.

7. Verwendung der Dibutene (14), hergestellt aus Feldbutanen, indem man
(a) die in den Feldbutanen (1) enthaltenen n- und iso-Butane in einer Dehydrierungsstufe (2) dehydriert,
(b) das Dehydrierungsgemisch (3) in einer Oligomerisierungsstufe (8) oligomerisiert, wobei man zwischen der Dehydrierungsstufe (2) und der Oligomerisierungsstufe (8) eine Selektivhydrierung (4) und/oder eine Reinigungsstufe (6) mit Molekularsieb in beliebiger Reihenfolge anordnet,
(c) aus dem Oligomerisierungsgemisch (9) das Restgas (12) abtrennt und, gegebenen falls nach Reinigung, in die Dehydrierungsstufe (2) zurückführt und
(d) aus den nach Abtrennung des Restgases (12) verbliebenen Oligomeren (11) das Dibuten (14) abtrennt,
zur Herstellung von Nonanolen durch Hydroformylierung und anschließende Hydrierung.

8. Verwendung der Di-n-Butene (17) und der restlichen Dibutene (18), hergestellt aus Feldbutanen, indem man
(a) die in den Feldbutanen (1) enthaltenen n- und iso-Butane in einer Dehydrierungsstufe (2) dehydriert,
(b) das Dehydrierungsgemisch (3) in einer Oligomerisierungsstufe (8) oligomerisiert, wobei man zwischen der Dehydrierungsstufe (2) und der Oligomerisierungsstufe (8) eine Selektivhydrierung (4) und/oder eine Reinigungsstufe (6) mit Molekularsieb in beliebiger Reihenfolge anordnet,
(c) aus dem Oligomerisierungsgemisch (9) das Restgas (12) abtrennt und, gegebenenfalls nach Reinigung, in die Dehydrierungsstufe (2) zurückführt,
(d) aus den nach Abtrennung des Restgases (12) verbliebenen Oligomeren (11) das Dibuten (14) abtrennt und
(e) die Dibutene (14) in einer Feindestillationsstufe (16) in Di-n-butene (17) und restliche Dibutene (18) trennt,
zur Herstellung von Nonanolen durch Hydroformylierung und anschließende Hydrierung.

9. Verwendung der Di-n-Butene (17), hergestellt aus Feldbutanen, indem man
(a) aus dem gegebenenfalls vorhydrierten Feldbutan (1) durch fraktionierte Destillation n-Butan (22) abtrennt und in die Dehydrierungsstufe (2) leitet, das verbleibende iso-Butan (23) in einer Isomerisierungsstufe (24) zu einem Gemisch von n-Butan und iso-Butan isomerisiert, aus dem Isomerisiserungsgemisch (25) durch fraktionierte Destillation das n-Butan abtrennt und zusammen mit dem direkt aus dem Feldbutan abgetrennten n-Butan (22) in die Dehydrierungsstufe (2) führt sowie das verbleibende iso-Butan (23) in die Isomerisierungsstufe (24) zurückführt,
(b) die n-Butane in einer Dehydrierungsstufe (2) dehydriert,
(c) das Dehydrierungsgemisch (3) in einer Oligomerisierungsstufe (8) oligomerisiert, wobei man zwischen der Dehydrierungsstufe (2) und der Oligomerisierungsstufe (8) eine Selektivhydrierung (4) und/oder eine Reinigungsstufe (6) mit Molekularsieb in beliebiger Reihenfolge anordnet,
(d) aus dem Oligomerisierungsgemisch (9) das Restgas (12) abtrennt und, gegebenenfalls nach Reinigung, in die Dehydrierungsstufe (2) zurückführt und
(e) aus den nach Abtrennung des Restgases (12) verschiedene Oligomeren (11) das Dibuten (14) abtrennt,
zur Herstellung von Nonanolen durch Hydroformylierung und anschließende Hydrierung.

10. Verwendung der Di-iso-Butene, hergestellt aus Feldbutanen, indem man
(a) aus dem gegebenenfalls vorhydrierten Feldbutan (1) durch fraktionierte Destillation iso-Butan abtrennt und in die Dehydrierungsstufe (2) leitet, das verbleibende n-Butan in einer Isomerisiserungsstufe (24) zu einem Gemisch von n-Butan und iso-Butan isomerisiert, aus dem Isomerisiserungsgemisch (25) durch fraktionierte Destillation das iso-Butan abtrennt und zusammen mit dem direkt aus dem Feldbutan abgetrennten iso-Butan in die Dehydrierungsstufe (2) führt sowie das verbleibende n-Butan in die Isomerisiserungsstufe (24) zurückführt,
(b) die iso-Butane in einer Dehydrierungsstufe (2) dehydriert,
(c) das Dehydrierungsgemisch 3 in einer Oligomerisierungsstufe 8 oligomerisiert, wobei man zwischen der Dehydrierungsstufe (2) und der Olgiomerisierungsstufe (8) eine Selektivhydrierung (4) und/oder eine Reinigungsstufe (6) mit Molekularsieb in beliebiger Reihenfolge anordnet,
(d) aus dem Oligomerisierungsgemisch (9) das Restgas (12) abtrennt und, gegebenenfalls nach Reinigung, in die Dehydrierungsstufe (2) zurückführt und
(e) aus den nach Abtrennung des Restgases (12) verbliebenen Oligomeren (11) das Dibuten (14) abtrennt
zur Herstellung von Nonanolen durch Hydroformylierung und anschließende Hydrierung.

11. Verwendung der Dodecene (15), hergestellt aus Feldbutanen, indem man
(a) die in den Feldbutanen (1) enthaltenen n- und iso-Butane in einer Dehydrierungsstufe (2) dehydriert und
(b) das Dehydrierungsgemisch (3) in einer Oligomerisierungsstufe (8) oligomerisiert, wobei man zwischen der Dehydrierungsstufe (2) und der Oligomerisierungsstufe (8) eine Selektivhydrierung (4) und/oder eine Reinigungsstufe (6) mit Molekularsieb in beliebiger Reihenfolge anordnet,
zur Herstellung von Waschrohstoffen durch Hydroformylierung, Hydrierung der Hydroformylierungsprodukte und Oxethylierung der Hydrierungsprodukte.

## Claims

1. A process for preparing butene oligomers from field butanes, characterized in that
(a) the n-butanes and isobutanes present in the field butanes (1) are dehydrogenated in a dehydrogenation stage (2) and
(b) the dehydrogenation mixture (3) is oligomerized in an oligomerization stage (8), a selective hydrogenation (4) and/or a purification stage (6) with a molecular sieve being arranged in any sequence between the dehydrogenation stage (2) and the oligomerization stage (8).

2. A process according to claim 1, characterized in that the residual gas (12) is separated off from the oligomerization mixture (9) and, if appropriate after purification, is recirculated to the dehydrogenation stage (2).

3. A process according to claim 2, characterized in that the dibutene (14) is separated off from the oligomers (11) remaining after separating off the residual gas (12).

4. A process according to claim 3, characterized in that the dibutenes (14) are separated in a precision distillation stage (16) into di-n-butenes (17) and remaining dibutenes (18).

5. A process according to any of claims 1 and 2, characterized in that di-n-butene is produced exclusively by separating off n-butane (22) from the possibly prehydrogenated field butane (1) by fractional distillation and passing it into the dehydrogenation stage (2), isomerizing the remaining isobutane (23) in an isomerization stage (24) to give a mixture of n-butane and isobutane, separating off the n-butane from the isomerization mixture (25) by fractional distillation and conducting it into the dehydrogenation stage (2), together with the n-butane (22) separated off directly from the field butane, and recirculating the remaining isobutane (23) into the isomerization stage (24).

6. Process according to any of claims 1 and 2, characterized in that diisobutene is produced exclusively by separating off isobutane from the possibly prehydrogenated field butane (1) by fractional distillation and passing it into the dehydrogenation stage (2), isomerizing the remaining n-butane in an isomerization stage (24) to give a mixture of n-butane and isobutane, separating off the isobutane from the isomerization mixture (25) by fractional distillation and conducting it to the dehydrogenation stage (2), together with the isobutane separated off directly from the field butane, and recirculating the remaining n-butane into the isomerization stage (24).

7. The use of the dibutenes (14) prepared from field butanes by
(a) dehydrogenating in a dehydrogenation stage (2) the n-butanes and isobutanes present in the field butanes (1),
(b) oligomerizing the dehydrogenation mixture (3) in an oligomerization stage (8), a selective hydrogenation (4) and/or a purification stage (6) with molecular sieve being arranged in any sequence between the dehydrogenation stage (2) and the oligomerization stage (8),
(c) separating off the residual gas (12) from the oligomerization mixture (9) and, if appropriate after purification, recirculating it to the dehydrogenation stage (2) and
(d) separating off the dibutene (14) from the oligomers (11) remaining after separating off the residual gas (12),
for preparing nonanols by hydroformylation and subsequent hydrogenation.

8. The use of the di-n-butenes (17) and the residual dibutenes (18), prepared from field butanes, by
(a) dehydrogenating in a dehydrogenation stage (2) the n-butanes and isobutanes present in the field butanes (1),
(b) oligomerizing the dehydrogenation mixture (3) in an oligomerization stage (8), a selective hydrogenation (4) and/or a purification stage (6) with molecular sieve being arranged in any sequence between the dehydrogenation stage (2) and the oligomerization stage (8),
(c) separating off the residual gas (12) from the oligomerization mixture (9) and, if appropriate after purification, recirculating it to the dehydrogenation stage (2),
(d) separating off the dibutene (14) from the oligomers (11) remaining after separating off the residual gas (12) and
(e) separating the dibutenes (14) in a precision distillation stage (16) into di-n-butenes (17) and residual dibutenes (18),
for preparing nonanols by hydroformylation and subsequent hydrogenation.

9. The use of the di-n-butenes (17), prepared from field butanes, by
(a) separating off n-butane (22) from the possibly prehydrogenated field butane (1) by fractional distillation and passing it into the dehydrogenation stage (2), isomerizing the remaining isobutane (23) in an isomerization stage (24) to give a mixture of n-butane and isobutane, separating off the n-butane from the isomerization mixture (25) by fractional distillation and conducting it into the dehydrogenation stage (2), together with the n-butane (22) separated off directly from the field butane, and recirculating the remaining isobutane (23) into the isomerization stage (24),
(b) dehydrogenating the n-butanes in a dehydrogenation stage (2),
(c) oligomerizing the dehydrogenation mixture (3) in an oligomerization stage (8), a selective hydrogenation (4) and/or a purification stage (6) with molecular sieve being arranged in any sequence between the dehydrogeration stage (2) and the oligomerization stage (8),
(d) separating off the residual gas (12) from the oligomerization mixture (9) and, if appropriate after purification, recirculating it to the dehydrogenation stage (2) and
(e) separating off the dibutene (14) from the various oligomers (11) after separating off the residual gas (12),
for preparing nonanols by hydroformylation and subsequent hydrogenation.

10. The use of the diisobutenes prepared from field butanes by
(a) separating off isobutane from the possibly prehydrogenated field butane (1) by fractional distillation and passing it into the dehydrogenation stage (2), isomerizing the remaining n-butane in an isomerization stage (24) to give a mixture of n-butane and isobutane, separating off the isobutane from the isomerization mixture (25) by fractional distillation and conducting it into the dehydrogenation stage (2), together with the isobutane separated off directly from the field butane, and recirculating the remaining n-butane into the isomerization stage (24),
(b) dehydrogenating the isobutanes in a dehydrogenation stage (2),
(c) oligomerizing the dehydrogenation mixture (3) in an oligomerization stage (8), a selective hydrogenation (4) and/or a purification stage (6) with molecular sieve being arranged in any sequence between the dehydrogenation stage (2) and the oligomerization stage (8),
(d) separating off the residual gas (12) from the oligomerization mixture (9) and, if appropriate after purification, recirculating it to the dehydrogenation stage (2) and
(e) separating off the dibutene (14) from the oligomers (11) remaining after separating off the residual gas (12),
for preparing nonanols by hydroformylation and subsequent hydrogenation.

11. The use of the dodecenes (15) prepared from field butanes by
(a) dehydrogenating in a dehydrogenation stage (2) the n-butanes and isobutanes present in the field butanes (1) and
(b) oligomerizing the dehydrogenation mixture (3) in an oligomerization stage (8), a selective hydrogenation (4) and/or a purification stage (6) with molecular sieve being arranged in any sequence between the dehydrogenation stage (2) and the oligomerization stage (8),
for preparing detergent raw materials by hydroformylation, hydrogenation of the hydroformylation products and ethoxylation of the hydrogenation products.

## Revendications

1. Procédé de fabrication d'oligomères de butène à partir de butanes de champ, caractérisé en ce qu'
• a) on déshydrogénise dans une étape de déshydrogénation (2) les n- et isobutanes contenus dans les butanes de champ (1),
• b) on oligomérise le mélange de déshydrogénation (3) dans une étape d'oligomérisation (8) dans laquelle on dispose entre l'étape de déshydrogénation (2) et l'étape d'oligomérisation (8) une hydrogénation sélective (4) et/ou une étape de purification (6) à l'aide d'un tamis moléculaire dans n'importe quelle séquence.

2. Procédé selon la revendication 1,
caractérisé en ce qu'
on sépare du mélange d'oligomérisation (9) le gaz résiduel (12) et éventuellement après purification, on le ramène à l'étape de déshydrogénation (2).

3. Procédé selon la revendication 2,
caractérisé en ce qu'
on sépare des oligomères (11) qui demeurent après séparation du gaz résiduel (12), le dibutène (14).

4. Procédé selon la revendication 3,
caractérisé en ce qu'
on sépare les dibutènes (14) dans une étape de distillation fine (16), en di-n-butène (17) et en dibutènes résiduels (18).

5. Procedé selon l'une des revendications 1 à 2,
caractérisé en ce qu'
on produit exclusivement des di-n-butènes dans lesquels on sépare le n-butane (22) par distillation fractionnee du butane de champ (1) éventuellement préhydrogéné et on le conduit à l'étape de déshydrogénation (2), on isomérise l'isobutane (23) qui demeure à une étape d'isomérisation (24) en un mélange de n-butane et d'isobutane, on sépare du mélange d'isomérisation (25) le n-butane par distillation fractionnée et on le conduit conjointement avec le n-butane (22) séparé directement du butane de champ à l'étape de déshydrogénation (2) et on ramène l'isobutane (23) qui demeure, à l'étape d'isomérisation (24).

6. Procédé selon l'une des revendications 1 à 2,
caractérisée en ce qu'
• on produit exclusivement du di-isobutène, dans lequel on sépare l'isobutane du butane de champ par distillation fractionnée et on le conduit à une étape de déshydrogénation (2),
• on isomérise le n-butane qui demeure dans une étape d'isomérisation (24) en un mélange de n-butane et d'isobutane,
• on sépare l'isobutane du mélange d'isomérisation (25) par distillation fractionnée et
• on conduit conjointement à l'isobutane séparé directement du butane de champ, à l'étape de déshydrogénation (2) ainsi qu'on ramène le n-butane qui demeure à l'étape d'isomérisation (24).

7. Utilisation des dibutènes (24) produits à partir des butanes de champ, dans laquelle,
• a) on déshydrogénise les n- et isobutanes contenus dans les butanes de champ (1) dans une étape de déshydrogénation (2),
• b) on oligomérise le mélange de dèshydrogénation (3) dans une étape d'oligomérisation (8) pour laquelle on dispose entre l'étape de déshydrogénation (2) et l'étape d'oligomérisation (8), une hydrogénation sélective (4) et/ou une étape de purification (6) à l'aide d'un tamis moléculaire dans n'importe quelle séquence,
• c) on sépare le gaz résiduel (12) du mélange d'oligomérisation (9) et le cas échéant après purification, on le ramène à l'étape de déshydrogénation (2) et
• d) on sépare des oligomères (11) qui demeurent après séparation des gaz résiduels (12), le dibutène (14),
en vue de la fabrication de nonanols par hydroformylation et hydrogénation corrélative.

8. Utilisation des di-n-butènes (17) et des dibutènes (18) résiduels préparés à partir des butanes de champ dans laquelle,
• a) on déshydrogénise les n- et isobutanes contenus dans les butanes de champ (1) dans une étape de déshydrogénation (2),
• b) on oligomérise le mélange de déshydrogénation (3) dans une étape d'oligomérisation (8) dans laquelle on dispose entre l'étape de déshydrogénation (2) et l'étape d'oligomérisation (8), une hydrogénation sélective (4) et/ou une étape de purification (6) avec un tamis moléculaire dans n'importe quelle séquence,
• c) on sépare les gaz résiduels (12) du mélange d'oligomèrisation (9) et éventuellement après purification, on le ramène à l'étape de déshydrogénation (2),
• d) on sépare le dibutène (14) des oligomères (11) demeurés après séparation des gaz résiduels (12) et
• e) on sépare les dibutènes (14) dans une étape de distillation fine (16) en di-n-butène (17) et en dibutènes résiduels,
en vue de la fabrication de nonanols par hydroformylation et hydrogénation corrélative.

9. Utilisation des di-n-butènes (17) fabriqués à partir des butanes de champ,
dans laquelle
• a) on sépare le n-butane (22) du butane de champ éventuellement préhydrogéné, par distillation fractionnée et on le conduit au stade de déshydrogénation (2), on isomérise l'isobutane (23) restent dans une étape d'isomérisation (24) en un mélange de n-butans et d'isobutane, on sépare par distillation fractionnée le n-butane du mélange d'isomérisation (25) et on le conduit conjointement au n-butane (22) séparé directement du butane de champ à l'étape de déshydrogénation (2) ainsi qu'on ramène l'isobutane (23) qui demeure à l'étape d'isomérisation (24),
• b) on déshydrogène le n-butane dans une étape de déshydrogénation (2),
• c) on oligomérise le mélange de déshydrogénation (3) dans une étape d'oligomérisation (8) dans laquelle on dispose entre l'étape de déshydrogénation (2) et l'étape d'oligomérisation (8) une hydrogénation sélective (4) et/ou une étape de purification (6) avec un tamis moléculaire en n'importe quelle séquence,
• d) on sépare du mélange d'oligomérisation (9) les gaz résiduels et on le ramène éventuellement après purification, à l'étape de déshydrogénation (2) et
• e) on sépare le dibutène (14),des différents oligomères (11) après séparation,
en vue de la fabrication de nonanols par hydroformylation et hydrogénation corrélative.

10. Utilisation des di-isobutènes fabriqués à partir de butanes de champ dans laquelle
• a) on sépare l'isobutane du butane de champ (1) éventuellement préhydrogéné par distillation fractionnée et on le conduit a l'étape de déshydrogénation (2), on isomérise le n-butane qui demeure dans une étape d'isomérisation (24) en un mélange du n-butane et d'isobutane, on sépare l'isobutane du mélange d'isomérisation (25) par distillation fractionnée et on le conduit conjointement avec l'isobutane séparé directement du butane de champ, à l'étape de déshydrogénation (2) ainsi qu'on ramène le n-butane qui demeure à l'étape d'isomérisation (24),
• b) on déshydrogénise les isobutanes dans une étape de déshydrogénation (2),
• c) on oligomérise le mélange de déshydrogénation (3) dans une étape d'oligomérisation (8), pour laquelle on dispose entre l'étape de déshydrogénation (2) et l'étape d'oligomérisation (8) une hydrogénation sélective (4) et/ou une étape de purification (6) avec un tamis moléculaire dans n' importe quelle séquence,
• d) on sépare les gaz résiduels (12) du mélange d'oligomérisation (9) et le cas échéant on le ramène après purification à l'étape de déshydrogénation (2) et,
• e) on sépare le dibutène (14) des oligomères (11) qui demeurent après séparation des gaz résiduels (12)
en vue de la fabrication de nonanols par hydroformylation et hydrogénation corrélative.

11. Utilisation des dodécènes (15) fabriqués à partir des butanes de champ, dans laquelle ,
• a) on déshydrogénise les n- et isobutanes contenus dans les butanes de champ (1) dans une étape de déshydrogénation (2) et,
• b) on oligomérise le mélange de déshydrogénation (3) dans une étape d'oligomérisation,
dans laquelle
on interpose entre l'étape de déshydrogénation (2) et l'étape d'oligomérisation (8) une hydrogénation sélective (4) et/ou une étape de purification avec un tamis moléculaire dans n'importe quelle séquence,
en vue de la fabrication de matières premières de lavage par hydroformylation, hydrogénation des produits d'hydroformylation et oxyéthylation des produits d'hydrogénation.
